# EUROPEAN PATENT APPLICATION

(11) **EP 2 116 252 A1**
(43) Date of publication of application: **11.11.2009**
(21) Application number: 08714821.9
(22) Date of filing: 15.02.2008
(51) Int. Cl.: A61K 36/296, A61K 31/7048, A61P 25/00, A61P 25/04, A61P 25/14, A61P 25/16, A61P 25/28, A61P 25/32

(54) **THE USE OF EPIMEDIUM FLAVONES AND EFFECTIVE COMPONENTS THEREOF FOR THE PREPARATION OF MEDICAMENTS OF PROMOTING PROLIFERATIONS AND DIFFERENTIATIONS OF NERVE CELLS**

(30) Priority: 16.02.2007 CN 200710078945
(71) Applicant: Xuanwu Hospital Of Capital Medical University, Xuanwu Beijing 100053 (CN)
(72) Inventor: LI, Lin, Xuanwu Beijing 100053 (CN); ZHANG, Lan, Xuanwu Beijing 100053 (CN); WEI, Haifeng, Xuanwu Beijing 100053 (CN); YAO, Ruiqin, Xuanwu Beijing 100053 (CN); LI, Xiaoli, Xuanwu Beijing 100053 (CN); CHU, Jing-long, Xuanwu Beijing 100053 (CN)
(74) Representative: Larcher, Dominique
(86) International application number: PCT/CN2008/000353
(87) International publication number: WO 2008/101412

(57) **Abstract**

The present invention discloses the use of epimedium flavanoide and its effective ingredient icarrin for the preparation of a medicament for promoting the proliferation and/or differentiation of neural cells and a method for treating diseases associated with the proliferation and differentiation of neural cells by using the same.

## Description

### Technical Field

The present invention relates to the use of epimedium flavanoide and its effective ingredient icariin for the preparation of a medicament for promoting proliferation and differentiation of neural cell, and to a method for treating nervous system diseases by using said medicament. Another aspect of the present invention relates to a pharmaceutical composition comprising epimedium flavanoide or its effective ingredient for treating or preventing nervous system diseases.

### Background Art

Neural stem cells (NSC) mean multi-potential cells derived from central nervous system or capable of differentiating into mature neural cells in central nervous system. Neural stem cells have self-renewal capability and multi-differentiation potency, and can differentiate into neurons, oligodendrocytes and astrocytes of central nervous system. Daughter cells generated by neural stem cells play an important role in the development and maintainance of central nervous system and practice of cell replacement therapy. Meanwhile, a great amount of experiments in vitro and in vivo have verified that NSC are present in brain in adult animals, these cells are in resting state at normal circumstance; under the stimulation of growth factors or pathological states, etc. these cells can proliferate, migrate and differentiate, new born neurons can substitute lost neural cells and produce a marked function. The finding of neural stem cells and the success in isolation and culture of neural stem cell in vitro broaden a road for cell replacement therapy of degenerative diseases of nervous system such as Parkinson's disease (PD), Alzheimer's disease (AD), Huntington's disease (HD), amyotrophic lateral sclerosis (ALS), etc., and can remarkably improve symptoms. Besides, central nervous system damage resulting from various causes such as ischemic brain or spinal cord damage, brain or spinal cord surgical trauma can lead to the reduction of the number of neural cells. Therefore, studies of an effective method for promoting the proliferation and differentiation of neural stem cells provide important means to treat neuron loss diseases caused by central nervous system damage and so on.

Epimedium is a traditional Chinese medicine for invigorating the kidney. The inventor has surprisingly found that epimedium flavanoide and its effective ingredient icarrin has good action to promote the proliferation and differentiation of neural stem cells, and thus accomplished this invention.

### Summary of the Invention

The present invention provides the use of epimedium flavanoide and its effective ingredient icarrin for the preparation of a medicament for promoting the proliferation and differentiation of neural stem cells.

In the present invention, the amount of icarrin in epimedium flavanoide is 40-90%, e.g. 60%. Icarrin (C₃₃H₄₀O₁₅, 3-((6-deoxymannopyranosyl)oxo)-7-(pyranoglucosyloxo)-5-hydroxy-2-(4-methoxyphen yl)-8-(3-methyl-2-butenyl)-4H-1-benzopyran-4-one; molecular weight: 674.69), its structure is as follows: 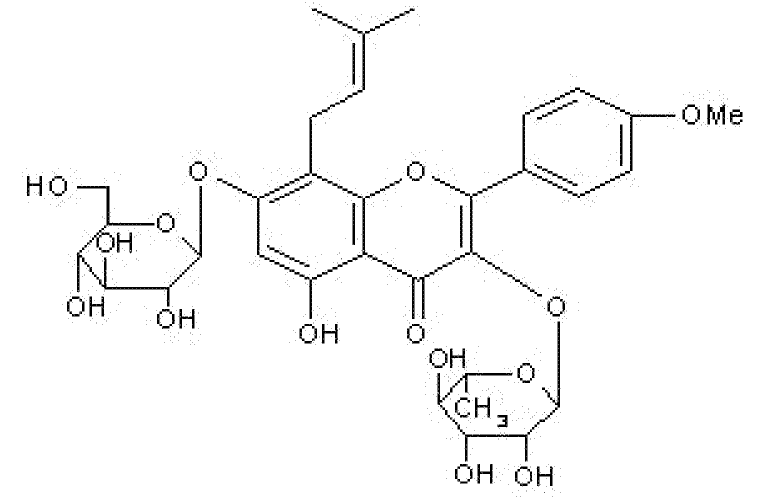 Epimedium flavanoide and icarrin are commercially available. They can be used for promoting the proliferation and differentiation of neural stem cells, particularly for the treatment of diseases associated with neuron loss resulting various causes, including degenerative diseases of nervous system, containing but being not limited to Parkinson's disease, Alzheimer's disease, Huntington's disease, amyotrophic lateral sclerosis , movement disorder diseases, spinal cord derived muscular atrophy and associated diseases, systemic astrophy disease affecting nervous system, basal nuclei degenerative diseases, dysmyotonia or primary extracorticospinal tract diseases; central nerve damage, containing but being not limited to ischemic, traumatic, and alcoholic brain or spinal cord damage. According to the present invention, said diseases are treated by repairing neurons. The present invention is also useful for the prevention of said diseases.

Another aspect of the present invention provides a pharmaceutical composition comprising epimedium flavanoide and/or icarrin, and a method for treatment or prevention of nervous system diseases by using said pharmaceutical composition.

With rapid renovation progress of modern molecular biology, a technology for proliferation and differentiation of mature neural cells which have grown well may be developed. Since epimedium flavanoide/icarrin promotes the proliferation and differentiation of neural stem cells, epimedium flavanoide/icarrin may have an action to promote the proliferation and differentiation of mature neural cells, and thereby treat nervous system diseases characterized by neuron loss resulting from various causes.

The method and use of epimedium flavanoide and/or icarrin mentioned above can be utilized separately to promote the proliferation and differentiation of stem cells in nervous system in a patient suffering from neural diseases, or by migrating extraneous stem cells into the nervous system in a patient suffering from neural diseases to proliferate and differentiate so as to achieve the purpose of treating or preventing nervous system diseases.

The epimedium flavanoide and/or icarrin according to the present invention can be mixed by conventional technical means with a pharmaceutically acceptable carrier into a pharmaceutical composition or various routine dosage forms, and administered by conventional means. Suitable administration dosage can be determined by a doctor based on the nature of diseases, the extent of the diseases, the age and body weight of patient, etc. factors.

Therefore, the present invention also relates to the use of icarrin analogues for the treatment of diseases characterized with neuron loss resulting from various causes. Icarrin analogues mean molecules having similarity to the substantive structure of icarrin, for example, molecules formed after the chemical groups of icarrin are protected with protective groups. For instance, derivatives in which hydroxyl of icarrin is substituted by C1-6 alkyl or halogen, and esters formed by icarrin with a pharmaceutically acceptable organic acid such as acetic acid or inorganic acid such as hydrochloric acid. Suitable protective groups are known in the art.

The components of the composition can be obtained from any suitable sources. For example, they can be in purified form or in the form of herbal medicines, or preferably in the form of herbal extracts, or in the form of herbal horticultural or vegetable equivalents, or in the form of chemical or functional equivalents of herbal extracts, and can be obtained by formulation of several chemical synthetics.

The composition of the present invention is estimated to oral administer, although it can be administered in any suitable way, such as intravenously, intranasally, intraperitoneally, subcutaneously, muscle, topically, suppository or implantation (controlled release molecules).

The pharmaceutically acceptable form of the composition can be a form suitable for injection, for example, a sterile aqueous solution and a sterile powder for temporarily preparing sterile injection solution or dispersion solution.

The composition must be stable at the conditions of preparation and storage, and should be stored under the condition of resistance to contamination of microorganism such as bacterium and fungi. Carrier can be a solvent or dispersion matrix, such as water, ethanol, polyols (e.g. glycerol, propanediol, and liquid polyethylene glycol), and their suitable mixtures and vegetable oils. It can maintain suitable flowability, e.g. by applying coat such as lecithin. Various antibacterial agents and antifungal agents, such as p-hydroxybenzoates, chlorobutanol, phenol, sorbic acid, merthiolate, can prevent microorganism. Under many conditions, the composition preferably comprises isosmotic substances, such as sugar or sodium chloride. Use of a delayed action absorber such as aluminum monostearate and gelatin in the composition can result in the delayed absorption of composition for injection.

The preparation of sterile injection solution comprises adding an active compound in a required amount to a suitable solvent having various other ingredients listed above, and then filtering and sterilizing. For sterile powders for preparing sterile injection solution, preferred preparation method comprises vacuum drying and freeze drying techniques to obtain powders of active ingredients, and adding any additional required ingredients of the previous sterile filtered solutions.

The composition can be administered orally, for example, administered together with an inert diluent or an assimilable edible carrier, or it can be encapsulated in gelatin capsule of hard or soft case, or it can be pressed into tablets, or it can be in the form of powders or directly added to dietary foods. For oral administration for treatment and/or prevention, the active compound can be applied by adding an excipient and in the form of absorbable tablets, buccal tablets, lozenges, capsules, elixirs, suspensions, syrups, glutinous rice paper sachets.

Broad dosage range can be applied depending on patients, the extent of patient's conditions, and path and medium for administration. In the composition having therapeutic action the amount of active compound is a suitable dosage. According to the preferred composition prepared in the present invention, its oral dosage unit form contains about 0.01 µg to about 2000 mg active compound. Optional amount includes about 1.0 µg to about 1500 mg, about 1 µg to about 1000 mg, and about 10 µg to about 500 mg.

Tablets, lozenges, pellets, capsules can also contain ingredients listed as follows: a binder such as gum, gum Arabic, corn starch or gelatin; an excipient such as dicalcium phosphate; a disintegrating agent such as corn starch, potato starch, alginic acid; lubricant such as magnesium stearate; and sweetening agent such as sucrose, lactose or saccharin or flavouring agent such as mint, wintergreen oil or cherry flavouring agent. When dosage unit form is capsule, it can contain a liquid carrier in addition to aforesaid types of substances. Various other substances can be present, such as coats or other physical forms for modifying dosage unit. For example, tablets, pellets or capsules can be coated with shellac, sugar or both. Syrups or elixirs can contain active compounds, sucrose as sweetening agent, methyl parabens and propyl parabens as preservative, pigments and flavoring agents such as cherry or orange flavoring agent. Certainly, any substance for preparing any dosage unit form should be pharmaceutically pure and substantially nontoxic in application amount. Further, the active compounds can be added into release-controlled formulations.

Pharmaceutically acceptable carriers and/or diluents include any and all solvents, dispersion media, coats, antibacterial agents and antifungal agents, isosmotic and absorption-delayed substances. These media and substances are known in the art to the use of pharmaceutically active substances. Any conventional media or substances other than those which are incompatible with active substances can be applied in the composition for treatment. Supplementary active ingredients can also be added in the composition.

The preparation of composition for parenteral administration in dosage unit form is particularly beneficial for the convenience of administration and the uniformity of dose. The dosage unit form used in the present invention means physical discrete unit suitable for use as a single dosage of treated mammal patients; a unit contains a calculated predetermined amount of an active substance and required pharmaceutically acceptable carriers to produce desirable therapeutic action.

Main active ingredients or ingredient groups are formulated with an effective amount of suitable pharmaceutically acceptable carries in a dosage unit form for convenient and effective administration. A dosage unit form, e.g. 100 g can contain 0.01 µg to about 2000 mg main active compounds. Expressed in ratio, said active compounds are generally present in about 0.5 µg to about 2000 mg/ml carriers. In the composition comprising supplementary active ingredients, the dosage is determined by reference of common dosage and the manner to administer said ingredients. Or the administration dosage can be raised in the form of the amount per kilogram body weight. Under this circumstance, per kilogram body weight can be administered with about 0.001 µg to about 1000 mg. The preferred range considered in the present invention includes 50 µg to 500 mg per kilogram body weight or about 0.01 µg to about 500 mg per kilogram body weight or about 0.1 µg to about 250 mg per kilogram body weight.

### Description of the Drawings

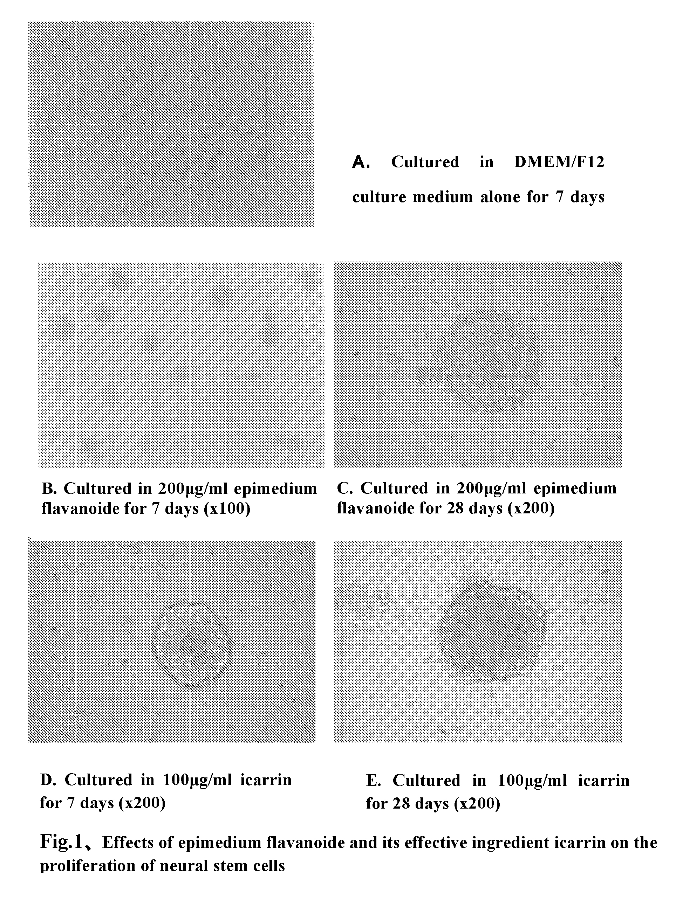

Fig. 1: Effect of epimedium flavanoide and its effective ingredient icarrin on the proliferation of neural stem cells. A. control group: cultured in DMEM/F12 culture solution without growth factors for 7 days, a majority of cells died (x200); B. cultured in DMEM/F12 culture solution without growth factors but with 200µg/ml epimedium flavanoide added for 7 days, many floating neurospheres were observed (x200); C. cultured in DMEM/F12 culture medium without growth factors but with 200µg/ml epimedium flavanoide added for 28 days, the neurospheres were still live, and their volume further increased (x200); D. cultured in DMEM/F12 culture medium without growth factors but with 100µg/ml icarrin added for 7 days, floating neurospheres were observed (x200); E. cultured in DMEM/F12 culture medium without growth factors but with 100µg/ml icarrin added for 28 days, the neurospheres still grew well, and their volume further increased (x200).

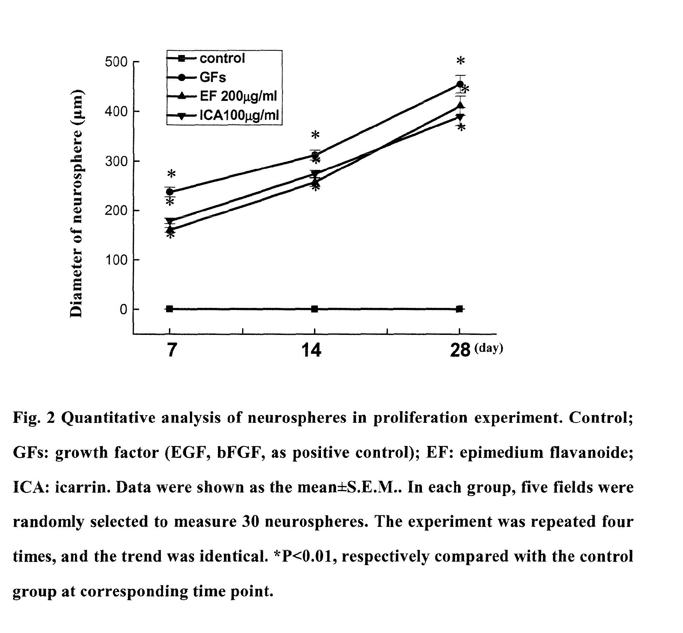

Fig. 2: The diameter of the neurospheres in the experiment of Fig. 1 was quantitatively analyzed. As can be seen, under the conditions without serum and growth factors, epimedium flavanoide and icarrin both led to the diameter of neurospheres increased gradually as the incubation time prolonged, and exhibited significant difference as compared with control group at each time point (p<0.01).

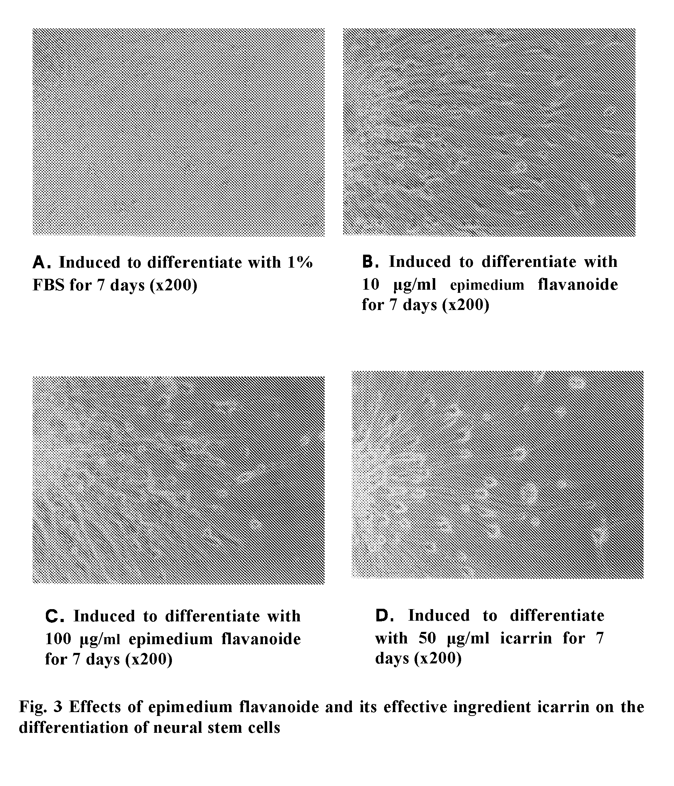

Fig. 3: Effects of epimedium flavanoide and its effective ingredient icarrin on the differentiation of neural stem cells. A. control group: the neural stem cells were induced to differentiate in DMEM/F12 culture medium containing 1% fetal bovine serum (FBS) for 7 days, unicells were emigrated from the edge of neurospheres, the shapes of emigrated cells were approximately divided into two types: (1) a small part of cells had a cell body morphology close to ellipse, and had 2-3 slender processes, which were similar to neurons in shape; (2) a majority of cells had larger cell body, and their morphology was irregular, similar to glial cells (x200); B. the neural stem cells were induced to differentiate in DMEM/F12 culture medium containing 10 µg/ml epimedium flavanoide (without FBS) for 7 days, many unicells were emigrated from neurospheres, the cells grew well, and a majority of the cells were similar to neurons in morphology (x200); C. the neural stem cells were induced to differentiate in DMEM/F12 culture medium containing 100 µg/ml epimedium flavanoide (without FBS) for 7 days, the growth state of the cells was similar to the group B, the neurospheres almost completely adhered, and differentiated better, and the proportion of the cells with elliptic cell body increased, and it was observed that the processes of this kind of cells became thick and long, the branches increased, and obvious axons and dendrites appeared, being typical neuron morphology (x200); D. the neural stem cells were induced to differentiate in DMEM/F12 culture medium containing 50 µg/ml icarrin (without FBS) for 7 days, the neurospheres grew well and almost completely adhered, and differentiated well, similar to the group C (x200).

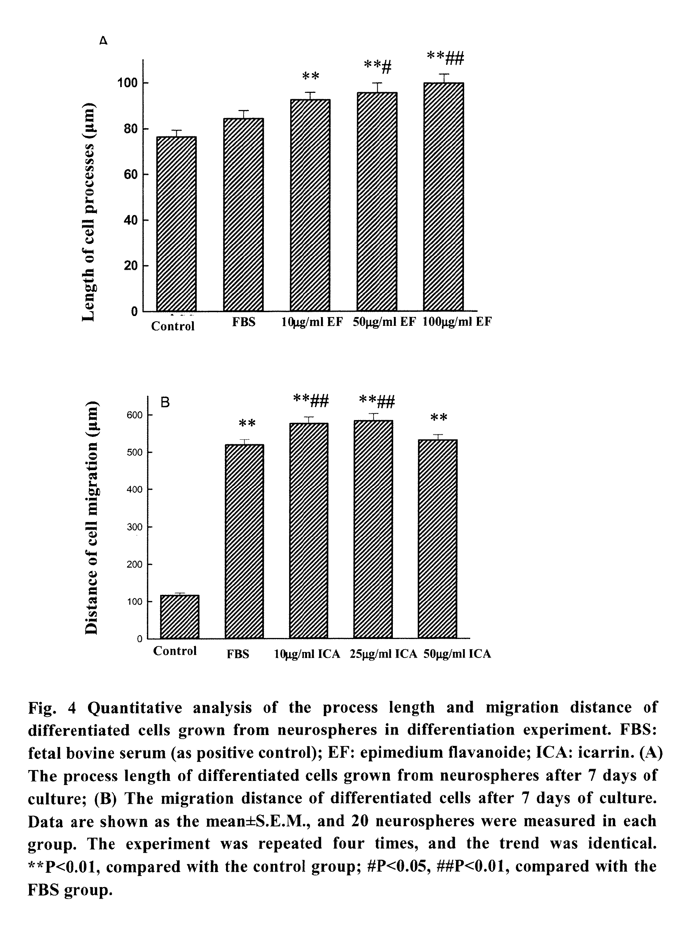

Fig. 4: The differentiated cells grown from the neurospheres in the experiments of Fig. 3 were quantitatively analyzed. It was observed that under the condition without fetal bovine serum, epimedium flavanoide and icarrin both led to longer processes of differentiated cells grown from the neurospheres, and farther distance of cell migration (P<0.01).

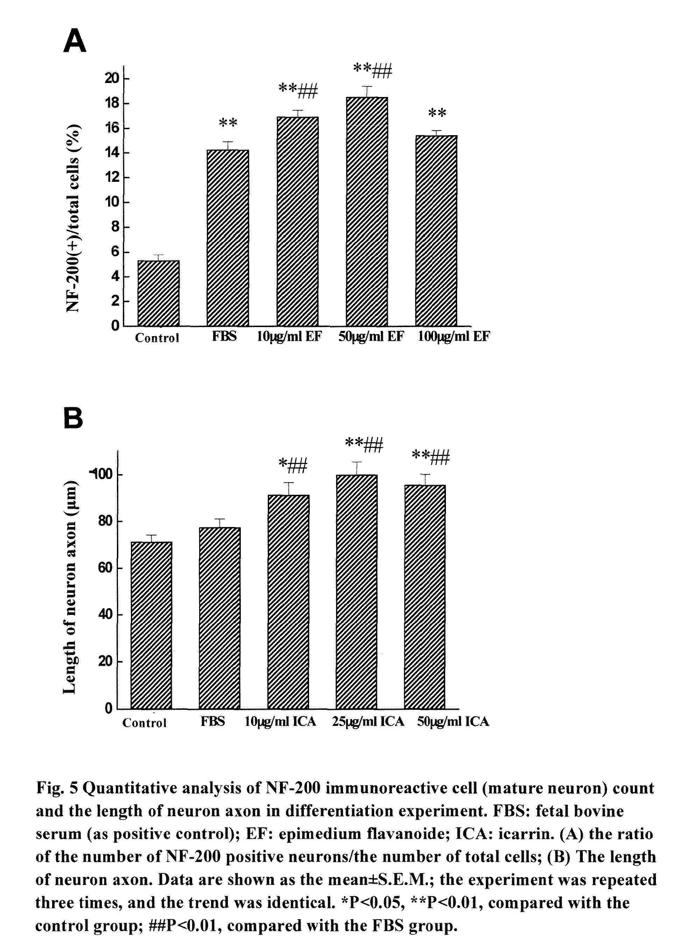

Fig. 5: The mature neurons differentiated from the neurospheres in the experiments of Fig. 3 were quantitatively analyzed. It was observed that under the condition without fetal bovine serum, epimedium flavanoide and icarrin both led to the number of NF-200 positive cells (mature neurons) increased, and the length of axon of neurons longer (P<0.01).

### Example 1

Epimedium flavanoide and its effective ingredient icarrin promote proliferation of neural stem cells

### 1. Purpose of the experiment

To observe the effects of epimedium flavanoide and its effective ingredient icarrin on the survival and proliferation of primary cultured neural stem cells (NSC).

### 2. Experimental methods

The brains were taken from neonatal 1 day SD rats under a sterile condition, and the hippocampi were isolated and placed in culture dish containing a small amount of high glucose DMEM/F12; the meninges and blood vessels were divested, and the tissue was cut into small pieces, added with 5 ml cell culture medium, lightly blown and beaten into cell suspension with flame polished Pasteur sucker, and filtered by 400 screen mesh. The ratio of living cells was counted by trypan-blue staining. After the counting, the cells were placed into a 25 ml culture flask at a density of 2x10⁶ cells/ml. Basal culture medium DMEM/F12 (1:1), cofactor B27(2%) and growth factor EGF (20ng/ml)+bFGF (10ng)/ml) were needed for conventional culture of NSC, and the cells grew as floating spheres in the culture medium. In order to observe the effect of different concentrations of epimedium flavanoide and its effective ingredient icarrin on primary cultured NSC, basal culture medium DMEM/F12 (1:1) and B27(2%) were used without growth factor, and epimedium flavanoide or icarrin at a final concentration of 10, 50, 100, 200, 400 µg/ml was added respectively. Cultures were maintained at 37 □ in a humidified atmosphere of 95% air/5% CO₂. The growth state of the cells was observed under inverted microscope, and the diameter of the neurospheres was measured by micro ruler.

The identification of neural stem cells: the neurospheres were taken, the culture medium containing growth factor EGF and bFGF were removed, DMEM/F12 containing 10% FBS was added, and inoculated in 24-well culture plates covered with polylysine. After 16 hours of culture, nestin and doublecortin (DCX) immunocytochemical staining was performed. The results showed that almost all cells were nestin positive and doublecortin positive, indicating that the cultured neurospheres were neural stem cells.

### 3. Experimental results

It was seen from Fig. 1 that when cells were cultured in DMEM/F12 culture medium containing no growth factors for 7 days, a majority of the cells died, and the cells were in diffused state. When cells were cultured in DMEM/F12 culture medium containing epimedium flavanoide or icarrin (without growth factors) for 7 days, many floating neurospheres were formed; the higher the concentration of agents was, the better the cells grew (the growth of the cells in 200-400 µg/ml group was the best); a number of neurospheres with large volume were observed. When cells were cultured for 28 days, the neurospheres in 200-400 µg/ml epimedium flavanoide groups and in 100-200 µg/ml icarrin groups not only still grew well, but also became significantly larger in volume (Fig. 1).

The quantitative analyses were conducted to the above experiments. It was shown that under the condition without serum and growth factor, epimedium flavanoide and icarrin caused the diameter of neurospheres increased, exhibiting significant difference as compared with the control groups at each time point (7, 14, 28 days) (P<0.01, Fig. 2).

### 4. Conclusion

Epimedium flavanoide and its effective ingredient icarrin have the effect similar to growth factors and can promote the proliferation of neural stem cells.

### Example 2

Epimedium flavanoide and its effective ingredient icarrin promote differentiation of neural stem cells

### 1. Purpose of the experiment

To observe the effects of epimedium flavanoide and its effective ingredient icarrin on the differentiation of neural stem cells.

### 2. Experimental methods

The second generation of neurospheres in culture was taken, and the culture medium containing growth factors in the culture flask was removed. The neurospheres were randomly divided into two groups: (1) fetal bovine serum (FBS) control group: the culture medium was DMEM/F12 + 1%FBS; (2) epimedium flavanoide treatment group: the culture medium was DMEM/F12 (without FBS) added with epimedium flavanoide at a final concentration of 10, 50, 100, 200 and 400µg/ml, respectively; (3) icarrin treatment group: the culture medium was DMEM/F12 (without FBS) added with icarrin at a final concentration of 10, 50, 100, 200 and 400µg/ml, respectively. The three groups of the cells were inoculated in 24-well culture plates covered with polylysine. After 7 days of culture, the morphology of the cells was observed under inverted microscope, and fluorescent immunostaining was performed and photographed.

### 3. Experimental results

It was seen from Fig. 3 that when the neural stem cells were induced to differentiate in DMEM/F12 culture medium containing 1% fetal bovine serum for 7 days, the neurospheres were completely adhered, unicells were emigrated from the edge of the neurospheres, and the shapes of emigrated cells were approximately divided into two types: (1) a small part of cells had a cell body morphology close to ellipse, and had 2-3 slender processes, being in the morphology of neurons; (2) a majority of the cells had larger cell body, and their morphology was irregular, many processes were observed, being in the morphology of glial cells. The results of immunocytochemical detection showed that the cells differentiated from the neurospheres were neurons, oligodendrocytes and astrocytes, indicating that these neural stem cells had poly differentiation potency.

When the neural stem cells were induced to differentiate in DMEM/F12 culture medium containing 10, 50, 100 µg/ml epimedium flavanoide (without FBS) for 7 days, many unicells were emigrated, the cells grew well, and the morphology of the cells were similar to that of FBS group. But the differences from FBS group were that the ratio of the neuron-like cells with elliptic cell body was increased, the processes of this kind of cells became thick and long, the branches increased, and obvious axons and dendrites appeared, being typical neuron shape. When the neural stem cells were induced to differentiate with 50, 100µg/ml icarrin for 7 days, the neurospheres grew well, almost completely adhered, and differentiated well, similar to the group treated by epimedium flavanoide ( Fig.3 ).

The quantitative analyses were conducted to the above experiments, and showed that under the condition without fetal bovine serum, epimedium flavanoide and icarrin both led to longer processes of differentiated cells grown from the neurospheres, and farther distance of cell migration (P<0.01), and the state was better as compared with the fetal bovine serum group (Fig. 4).

NF-200 is an immunostaining marker of mature neuron and can verify the phenotype of differentiated neuron. Under the condition without fetal bovine serum, epimedium flavanoide and icarrin both caused the number of NF-200 positive cells increased, and the length of the axons of neurons longer (P<0.01), and the cell state was better as compared with fetal bovine serum group (Fig. 5).

### 4. Conclusion

Epimedium flavanoide and its effective ingredient icarrin have promotive effect on the differentiation of neural stem cells to neurons.

## Claims

1. Use of epimedium flavanoide and/or icariin or analogues thereof for the preparation of a medicament for promoting proliferation and/or differentiation of neural cells.

2. The use according to claim 1, wherein said promotion of proliferation and/or differentiation of neural cells is used for treating diseases associated with neuron loss resulting from various causes.

3. The use according to claim 1, wherein said promotion of proliferation and/or differentiation of neural cells is used for treating nervous system degenerative diseases.

4. The use according to claim 2 or 3, wherein said diseases are Parkinson's disease, Huntington's disease, amyotrophic lateral sclerosis , movement disorder diseases, spinal cord derived muscular atrophy and associated diseases, systemic atrophy disease affecting nervous system, basal nuclei degenerative diseases, dysmyotonia, primary extracorticospinal tract diseases.

5. The use according to claim 2 or 3, wherein said disease is Alzheimer's disease.

6. The use according to claim 1, wherein said promotion of proliferation and/or differentiation of neural cells is used for treating central nerve damage.

7. The use according to claim 6, wherein the central nerve damage is ischemic, traumatic, and alcoholic brain or spinal cord damage.

8. The use according to claim 1, wherein the active ingredient in the medicament is epimedium flavanoide.

9. The use according to claim 8, wherein the amount of icariin in epimedium flavanoide is 40-90% by weight.

10. The use according to claim 9, wherein the amount is 60% by weight.

11. The use according to claim 1, wherein the active ingredient in the medicament is I icariin.

12. A pharmaceutical composition for treating or preventing nervous system diseases, **characterized in that** it comprises epimedium flavanoide and/or icariin or analogues thereof.

13. A method for treating or preventing nervous system diseases, comprising administering a therapeutically effective amount of epimedium flavanoide and/or icariin or analogues thereof or a pharmaceutical composition comprising the same to a patient in need of this treatment.
